# EUROPEAN PATENT APPLICATION

(11) **EP 0 992 483 A1**
(43) Date of publication of application: **12.04.2000**
(21) Application number: 99307482.2
(22) Date of filing: 22.09.1999
(51) Int. Cl.: C07C 67/54, C07C 67/05

(54) **Process for purifying a process stream**

(30) Priority: 08.10.1998 GB 9821970
(71) Applicant: BP Chemicals Limited, London EC2M 7BA (GB)
(72) Inventor: Bristow, Timothy Crispin, Hull HU12 8DS (GB); Clarke, Robert William, Hull HU12 8DS (GB); Carr, Michael Peter, Ingleby Barwick, Stockton TS17 OYQ (GB); Kitchen, Simon James, Hull HU12 8DS (GB)
(74) Representative: Perkins, Nicholas David

(57) **Abstract**

In a process for removing ethyl acetate from a process stream comprising vinyl acetate, acetic acid, water and an ethyl acetate impurity, by the steps of: (a) introducing the process stream to a distillation column, (b) distilling the process stream to obtain a vinyl acetate-containing fraction at an upper end of the distillation column and an acetic acid-containing fraction at a lower end of said distillation column, and (c) withdrawing, a side stream comprising the ethyl acetate from an intermediate region of the distillation column, it has been found that by withdrawing the side stream as a vapour, the concentration of ethyl acetate obtainable from the distillation column is higher than the concentration of ethyl acetate obtainable by withdrawing the side stream as a liquid and is greater than 10 % by weight of the side stream.

## Description

The present invention relates to a process for purifying a process stream which comprises vinyl acetate.

Vinyl acetate may be produced by contacting acetic acid and ethylene with oxygen in the presence of a catalyst. The reaction produces a product stream comprising vinyl acetate, water and acetic acid, and an ethyl acetate impurity. These components may be separated in a distillation column. Typically, a near azeotropic mixture comprising vinyl acetate and water (bp ∼66°C) is removed from the top of the distillation column and a mixture comprising acetic acid and water (bp 125°C) is removed from the base of the column.

As a mid-boiling component (bp 77°C), the ethyl acetate impurity accumulates in a region between the top and bottom of the column. In a typical 60 tray column, ethyl acetate accumulates to appreciable concentrations between trays 8 and 20 (the bottom tray being numbered 1).

Conventionally, the ethyl acetate impurity is removed from the column as a side stream which is withdrawn from the column at or near tray 10. For convenience, the side stream is withdrawn as a liquid so as to avoid the need for complex condensation equipment that would be required for a vapour draw.

The liquid side stream comprises ethyl acetate as well as other components which are of commercial value such as vinyl acetate and acetic acid. The concentration of ethyl acetate in the side stream is low (e.g. about 2 to 7 mol. %) and, accordingly, a significant amount of vinyl acetate and acetic acid has to be withdrawn from the side of the distillation column to enable the concentration of ethyl acetate remaining in the purified vinyl acetate product to be reduced to desirably low levels (e.g. 10 to 500 ppm). Thus, for the conventional vinyl acetate purification process to be economically attractive the vinyl acetate and acetic acid in the side stream must be recovered and recycled back to the process for further purification. This is achieved by introducing the side stream into an additional distillation column which is operated at high reflux ratios (circa 10). The ethyl acetate is concentrated and removed, and the recovered materials are recycled to the main distillation column for further processing.

The cost of building and operating an additional distillation column is high, particularly because of the high reflux ratios required to concentrate the ethyl acetate present in the side stream.

It is among the objects of the present invention to increase the concentration of ethyl acetate in the side stream so as to eliminate or reduce the need for purifying the side stream by an additional distillation step.

This may be achieved by withdrawing the side stream in the vapour phase. We have found that by withdrawing a side stream as a vapour, the concentration of ethyl acetate obtainable from a distillation column is higher than the concentration of ethyl acetate obtainable by withdrawing the side stream as a liquid.

Accordingly, the present invention provides a process for removing ethyl acetate from a process stream comprising vinyl acetate, acetic acid, water and an ethyl acetate impurity, said process comprising the steps of:
a) introducing said process stream to a distillation column,
b) distilling said process stream to obtain a vinyl acetate-containing fraction at an upper end of the distillation column and an acetic acid-containing fraction at a lower end of said distillation column, and
c) withdrawing, in the vapour phase, a side stream comprising said ethyl acetate impurity at a concentration of greater than 10 % by weight of the side stream, from an intermediate region of said distillation column.

Preferably, the ethyl acetate impurity is withdrawn from a region of the distillation column where the concentration of ethyl acetate is at or near a maximum.

By removing the side stream as a vapour, a side stream having an ethyl acetate concentration which is higher than that observed with a conventional liquid side stream is obtained. For example, whereas an ethyl acetate concentration of about 2 to 7 mol. % is observed when a side stream is withdrawn from a column as a liquid, an ethyl acetate concentration of above 12 mol. % may be observed when a side stream is withdrawn from the column as a vapour. As a result of this increase in ethyl acetate concentration, the amount of other (valuable) process materials that must be withdrawn from the side of the distillation column in order to ensure that sufficient ethyl acetate is removed from the distillation column is less than that required using a conventional liquid draw. Accordingly, further processing of the side stream is not necessary and the side stream may be disposed of directly.

The process of the present invention may be carried out using any suitable distillation column. It will be understood that the present invention is not intended to be limited to the type of distillation column employed.

The process stream and ethyl acetate impurity may be introduced into the distillation column via an inlet located between the top and bottom of the column. Conditions in the distillation column may be controlled such that as distillation proceeds, a near azeotropic mixture of vinyl acetate and water is produced at the top of the column, and acetic acid and water is produced at the base of the column.

As a mid-boiling component, ethyl acetate impurity accumulates in an intermediate region of the column. For example, when a 60 tray column is used, the ethyl acetate concentration tends to be highest in the region of trays 6 to 20 numbered from the bottom. In the present invention, the ethyl acetate is removed as a vapour side stream from one or more of the intermediate trays of the column, preferably in the region of trays 6 to 20 numbered from the bottom, more preferably at or near trays 8 and/or 9 numbered from the bottom of a 60 tray column. Alternatively, the distillation column may have 72 actual trays and the side stream may be withdrawn from tray 10, 12 or 14 number from the bottom.

The side stream may be withdrawn at a temperature of between 80° and 120°C, preferably between 90° and 110°C, and most preferably between 93° and 105°C. It will be understood, however, that the temperature at which the side stream is withdrawn will vary depending on the pressure at which the distillation is carried out.

The distillation is preferably carried out at any suitable pressure.

The side stream produced by the present invention may have an ethyl acetate concentration of above 13 wt% and, preferably above 19 wt %. Expressed in mol. %, the ethyl acetate concentration of the side stream is preferably above 8 mol. %, and especially above 12 mol %. It will be understood, however, that the precise concentrations of ethyl acetate present in the side stream will vary depending on the initial concentration of ethyl acetate present in the process stream.

The process of the present invention may be used to purify a process stream from a vinyl acetate production process. The process stream comprises vinyl acetate, ethyl acetate, water and acetic acid. The process stream may also comprise dissolved ethylene, carbon dioxide, acetaldehyde and other gases, methyl acetate, vinyl propionate and a mixture of heavy organic compounds and corrosion metals. Polymerisation inhibitors may also be present in the process stream.

For certain applications, purification of the side stream is necessary. Accordingly, the side stream may be introduced to an additional distillation column to concentrate the ethyl acetate for removal. Because of the increased concentration of ethyl acetate, however, the additional column need not be operated at the high reflux ratios required for purifying conventional side streams that are withdrawn in the liquid phase. Preferably, reflux ratios of less than 10 are employed. Material recovered from this additional distillation column may be recycled to the distillation column of the present invention for reprocessing.

It has been found that the side stream may form two liquid phases when condensed - a water-rich aqueous phase and an ethyl acetate-rich organic phase which can be separated. Acetic acid tends to partition into the aqueous phase which may assist in its recovery from the side stream. Vinyl acetate tends to partition into the organic phase.

An embodiment of the present invention will now be described with reference to the following example.

### Example

The example is a computer simulation of a process carried out in accordance with an embodiment of the present invention. The example simulates the conditions encountered in a distillation column. The simulation is modelled on 43 theoretical stages which are numbered sequentially from bottom upwards. The simulation was performed with a pressure at the top of the distillation column of 1.1 bara.

Two feed streams (minor process recycle stream (1), process recycle stream (2)) are introduced into the distillation column at stage 42, and three feed streams (crude vinyl acetate containing feed stream (3), process recycle stream (4) and process recycle stream (5)) are introduced into the distillation column at stage 25.

In the distillation, the components of the feed streams are separated and collected from various locations along the column. A near azeotropic mixture of vinyl acetate and water is produced at the top of the column and a mixture consisting essentially of acetic acid and water is collected from the base of the column.

The overhead from the distillation column when passed through a condenser would be expected to form a condensate which forms two liquid phases and a vent gas. One of the liquid phases (organic phase) is returned in part as reflux to the head of the column (stream (6)), the remainder of the organic phase is drawn off at a rate of 498.625 kg/hr together with the second liquid phase (aqueous phase).

A partial condensation of the reboiler vapour was also included which resulted in a draw-off below stage 1 of 333.260 kg/hr.

The compositions of the streams are described in Table 1 below.

As a mid-boiling component, ethyl acetate accumulates in an intermediate region of the column. The ethyl acetate is removed from the side of the column as an output stream from stage no. 7. The output stream is withdrawn in the vapour phase and its characteristics are described in the Table 2 below.

**Table 1**

| components (kg/h) | Stream 1 | Stream 2 | Stream 3 | Stream 4 | Stream 5 | Stream 6 |
|---|---|---|---|---|---|---|
| | Minor process recycle | Process recycle stream | Crude vinyl acetate feed | Process recycle stream | Process recycle stream | Reflux |
| dissolved gases | - | - | 14.410 | 0.040 | 0.142 | 3.5413 |
| acetaldehyde | - | - | 2.223 | 0.107 x 10⁻² | - | 5.2664 |
| vinyl acetate | 2.500 | 50.849 | 448.413 | 0.022 x 10⁻² | 2.959 | 1868.0854 |
| ethyl acetate | - | 0.003 | 0.5 | 0.086 x 10⁻³ | 0.002 | 0.0694 |
| vinyl propionate | - | - | 0.070 | - | 0.077 x 10⁻² | - |
| water | - | 0.548 | 183.175 | 4.519 | 2.615 | 19.9572 |
| acetic acid | - | - | 1424.625 | 3.725 | 27.875 | - |
| ethylene diacetate | - | - | 0.0358 | - | 0.002 | - |
| polymerisation inhibitors | - | 0.065 | 0.162 | - | - | - |
| methyl acetate | - | 0.002 | 0.082 | - | 0.064 x 10⁻² | 0.2958 |
| total rate of flow (kg/h) | 2.500 | 51.468 | 2072.5 | 8.286 | 33.596 | 1897.2155 |
| total rate of flow (kmol/h) | 0.029 | 0.622 | 39.58 | 0.314 | 0.648 | 23.0296 |
| temperature | 25°C | 83°C | 107.9 | 31°C | 133°C | 35°C |
| pressure (bara) | 8 | 1.1 | 8 | 7.95 | 1.5 | 1.1 |

### Comparative Example

The procedure of the example above is repeated except that the output stream from tray number 7 is withdrawn in the liquid phase. The characteristics of this liquid output stream are described in Table 2 below.

**Table 2**

| components (kg/h) | example | comparative example |
|---|---|---|
| dissolved gases | - | - |
| acetaldehyde | - | - |
| vinyl acetate | 0.658 | 0.139 |
| ethyl acetate | 0.330 | 0.188 |
| vinyl propionate | 0.013 | 0.006 |
| water | 0.187 | 0.160 |
| acetic acid | 0.42 | 0.975 |
| ethylene diacetate | - | 0.017 x 10⁻³ |
| polymerisation inhibitors | - | 0.075 x 10⁻³ |
| methyl acetate | 0.092 x 10 ⁻² | 0.027 x 10⁻² |
| total rate of flow (kg/h) | 1.61 | 1.469 |
| total rate of flow (kmol/h) | 0.029 | 0.029 |
| wt % ethyl acetate | 20.5 | 12.8 |
| mol % ethyl acetate | 13.0 | 7.4 |
| temperature (°C) | 102.9 | 102.0 |
| pressure (bara) | 1.444 | 1.444 |

As can be seen from Table 2 above, the concentration of ethyl acetate in the output stream of the example is higher than the concentration of ethyl acetate in the comparative example. Thus, higher concentrations of ethyl acetate may be withdrawn from the distillation column when the output stream is withdrawn as a vapour rather than a liquid.

## Claims

1. A process for removing ethyl acetate from a process stream comprising vinyl acetate, acetic acid, water and an ethyl acetate impurity, said process comprising the steps of:
a) introducing said process stream to a distillation column,
b) distilling said process stream to obtain a vinyl acetate-containing fraction at an upper end of the distillation column and an acetic acid-containing fraction at a lower end of said distillation column, and
c) withdrawing, in the vapour phase, a side stream comprising said ethyl acetate impurity at a concentration of greater than 10 % by weight of the side stream, from an intermediate region of said distillation column.

2. A process as claimed in claim 1 wherein the side stream has an ethyl acetate concentration of above 13 % by weight.

3. A process as claimed in claim 1 wherein the side stream has an ethyl acetate concentration of above 19 % by weight.

4. A process as claimed in claim 1 wherein the side stream has an ethyl acetate concentration of above 8 mol %.

5. A process as claimed in claim 1 wherein the side stream has an ethyl acetate concentration of above 12 mol %.

6. A process as claimed in any one of the preceding claims wherein the distillation column has 60 trays and the side stream is withdrawn in the region of trays 6 to 20 numbered from the bottom.

7. A process as claimed in claim 6 wherein the side stream is withdrawn from one or more of the trays at or near trays 8 and/or 9 numbered from the bottom.

8. A process as claimed in any one of claims 1 to 5 wherein the distillation column has 72 actual trays and the side stream is withdrawn from tray 10, 12 or 14 number from the bottom.

9. A process as claimed in any one of the preceding claims wherein the withdrawn side stream is introduced into an additional distillation column to concentrate the ethyl acetate.

10. A process as claimed in claim 9 wherein the additional distillation column is operated with a reflux ratio of less than 10.

11. A process as claimed in any one of the preceding claims wherein the withdrawn side stream is condensed to form a water-rich phase and an ethyl acetate-rich phase which are separated.

12. A process for the production of vinyl acetate by contacting acetic acid and ethylene with oxygen in the presence of a catalyst to produce a product stream comprising vinyl acetate, water, acetic acid and an ethyl acetate impurity characterised in that the ethyl acetate impurity is removed from the product stream by a process as claimed in any one of the preceding claims.
